# EUROPÄISCHE PATENTANMELDUNG

(11) **EP 0 695 743 A1**
(43) Veröffentlichungstag der Anmeldung: **07.02.1996**
(21) Anmeldenummer: 95111580.7
(22) Anmeldetag: 24.07.1995
(51) Int. Cl.: C07D 253/075

(54) **Verfahren zur Herstellung von 1,2,4-Triazin-3,5-dionen**

(30) Priorität: 04.08.1994 DE 4427529
(71) Anmelder: BAYER AG, D-51368 Leverkusen (DE)
(72) Erfinder: Gallenkamp, Bernd, Dr., D-42113 Wuppertal (DE); Hallenbach, Werner, Dr., D-40789 Monheim (DE); Rohe, Lothar, Dr., D-42113 Wuppertal (DE)

(57) **Zusammenfassung**

Die Erfindung betrifft ein neues Verfahren zur Herstellung von 1,2,4-Triazin-3,5-dionen durch Decarboxylierung von entsprechenden 6-Carboxy-1,2,4-triazin-3,5-dionen, dadurch gekennzeichnet, daß man in aprotischen organischen Lösungsmitteln arbeitet.

## Beschreibung

Die Erfindung betrifft ein neues Verfahren zur Herstellung von 1,2,4-Triazin-3,5-dionen.

Es ist bekannt, daß 1,2,4-Triazin-3,5-dione durch Decarboxylierung von entsprechenden 6-Carboxy-1,2,4-triazin-3,5-dionen hergestellt werden können (vgl. DE-A 2423972; DE-A 2722537; EP-A 330041; J. Heterocycl. Chem. 17 (1980). 1365-1368).

Hierbei werden im allgemeinen Lösungsmittel mit sehr hohen Siedepunkten, wie z.B. Diphenylether, und/oder gegebenenfalls katalytisch wirkende Stoffe, wie z.B. Thioharnstoff oder Mercaptoessigsäure, verwendet. Die Decarboxylierungsprodukte werden in vielen Fällen in unbefriedigender Qualität erhalten, wobei insbesondere die Abtrennung von Lösungsmittel und unerwünschten Zersetzungsprodukten problematisch ist. Wenn die Decarboxylierung ohne Lösungsmittel durchgeführt wird, fallen die Produkte in schwer verarbeitbarer Form an.

Es wurde nun gefunden, daß man 1,2,4-Triazindione der allgemeinen Formel (I) 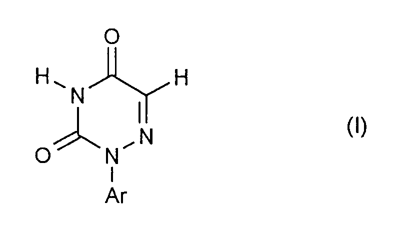
in welcher
- Ar: für jeweils gegebenenfalls substituiertes Aryl oder Heteroaryl steht,
in guten Ausbeuten und in hoher Reinheit erhält, wenn man 6-Carboxy-1,2,4-triazindione der allgemeinen Formel (II) 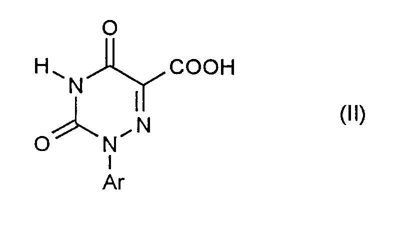
in welcher
- Ar: die oben angegebene Bedeutung hat,
in aprotischen organischen Lösungsmitteln - ohne weitere Zusatzstoffe - mit Siedepunkten zwischen 50°C und 150°C, bei Temperaturen zwischen 80°C und 220°C, gegebenenfalls unter erhöhtem Druck umsetzt.

Überraschenderweise können nach dem erfindungsgemäßen Verfahren die 1,2,4-Triazin-3,5-dione der allgemeinen Formel (I) bei glatter Umsetzung in guten Ausbeuten und in hoher Reinheit erhalten werden, wobei die oben genannten Nachteile der bekannten Herstellungsmethoden vermieden werden.

Bei Durchführung des erfindungsgemäßen Verfahrens unter erhöhtem Druck können auch relativ niedrig siedende Lösungsmittel verwendet werden. Dadurch wird die thermische Belastung von Edukt und Produkt verringert und die Aufarbeitung (mit destillativer Rückgewinnung des Lösungsmittels) vereinfacht.

Das erfindungsgemäße Verfahren stellt somit eine wertvolle Bereicherung des Standes der Technik dar.

Das erfindungsgemäße Verfahren betrifft vorzugsweise die Herstellung von Verbindungen der Formel (I), in welcher
- Ar: für jeweils gegebenenfalls substituiertes Phenyl, Naphthyl, Pyridyl, Pyrimidyl, Pyrazinyl, Pyridazinyl oder Triazinyl steht, wobei die möglichen Substituenten vorzugsweise ausgewählt sind aus der Reihe Halogen, Hydroxy, Amino, Formyl, Cyano, Nitro, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, C₁-C₄-Alkylamino, Di-(C₁-C₄-alkyl)-amino, C₁-C₄-Alkyl-carbonyl, C₁-C₄-Alkoxy-carbonyl, Phenyl, Phenoxy, Phenylthio, Phenylsulfinyl, Phenylsulfonyl, Phenylamino, Phenylcarbonyl, Phenylhydroxymethyl, Phenyl-cyanomethyl, Phenyl-C₁-C₄-alkyl, Pyridyl, Pyrimidinyl, Pyridyloxy, Pyrimidyloxy, Pyridylthio, Pyrimidylthio, Pyridylamino, Pyrimidylamino (wobei die Phenyl-, Pyridyl-, oder Pyrimidyl-Gruppierungen jeweils gegebenenfalls durch Halogen, Hydroxy, Amino, Formyl, Cyano, Nitro, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, C₁-C₄-Alkylamino, Di-(C₁-C₄-alkyl)-amino, C₁-C₄-Alkyl-carbonyl und/oder C₁-C₄-Alkoxy-carbonyl substituiert sind).

Die Erfindung betrifft insbesondere die Herstellung von Verbindungen der Formel (I), in welcher
- Ar: für gegebenenfalls substituiertes Phenyl steht, wobei die möglichen Substituenten vorzugsweise ausgewählt sind aus der Reihe Fluor, Chlor, Brom, Hydroxy, Amino, Formyl, Cyano, Nitro, Methyl, Ethyl, n- oder i-Propyl, n-, i-, s- oder t-Butyl, Trifluormethyl, Methoxy, Ethoxy, Difluormethoxy, Trifluormethoxy, Methylthio, Ethylthio, Trifluormethylthio, Methylsulfinyl, Ethylsulfinyl, Trifluormethylsulfinyl, Methylsulfonyl, Ethylsulfonyl, Trifluormethylsulfonyl, Methylamino, Ethylamino, Dimethylamino, Acetyl, Propionyl, Methoxycarbonyl, Ethoxycarbonyl, Phenyl, Phenoxy, Phenylthio, Phenylsulfinyl, Phenylsulfonyl, Phenylamino, Phenylcarbonyl, Phenylhydroxymethyl, Phenyl-cyanomethyl, Benzyl, Pyridyl, Pyrimidyl, Pyridyloxy, Pyrimidyloxy, Pyridylthio, Pyrimidylthio, Pyridylamino, Pyrimidylamino (wobei die Phenyl-, Pyridyl- oder Pyrimidyl-Gruppierungen jeweils gegebenenfalls durch Fluor, Chlor, Brom, Hydroxy, Amino, Formyl, Cyano, Nitro, Methyl, Ethyl, n- oder i-Propyl, n-, i-, s- oder t-Butyl, Trifluormethyl, Methoxy, Ethoxy, Difluormethoxy, Trifluormethoxy, Methylthio, Ethylthio Trifluormethylthio, Methylsulfinyl, Ethylsulfinyl, Trifluormethylsulfinyl, Methylsulfonyl, Ethylsulfonyl, Trifluormethylsulfonyl, Methylamino, Ethylamino, Dimethylamino, Acetyl, Propionyl, Methoxycarbonyl und/oder Ethoxycarbonyl substituiert sind).

Verwendet man beispielsweise 6-Carboxy-2-(4-hydroxy-3-methylphenyl)-1,2,4-triazin-3,5(2H,4H)-dion als Ausgangsverbindung, so kann der Reaktionsablauf beim erfindungsgemäßen Verfahren durch das folgende Formelschema skizziert werden: 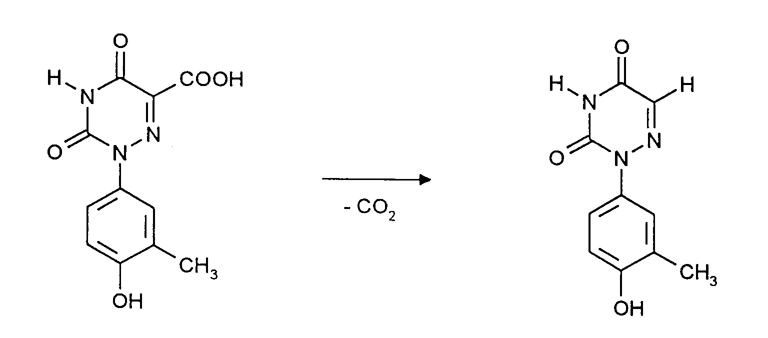
Die beim erfindungsgemäßen Verfahren zur Herstellung der Verbindungen der allgemeinen Formel (I) als Ausgangsstoffe zu verwendenden 6-Carboxy-1,2,4-triazindione sind durch die Formel (II) allgemein definiert. In der Formel (II) hat Ar vorzugsweise bzw. insbesondere diejenige Bedeutung, die bereits oben im Zusammenhang mit der Beschreibung der Verbindungen der Formel (I) als bevorzugt bzw. als insbesondere bevorzugt für Ar angegeben wurde.

Die Ausgangsstoffe der Formel (II) sind bekannt und/oder können nach an sich bekannten Verfahren hergestellt werden (vgl. DE-A 2423972; DE-A 2722537; EP-A 330041; J. Heterocycl. Chem. 17 (1980), 1365-1368).

Das erfindungsgemäße Verfahren wird unter Verwendung von einem oder mehreren aprotischen organischen Lösungsmitteln durchgeführt. Hierzu gehören insbesondere aliphatische, alicyclische oder aromatische, gegebenenfalls halogenierte Kohlenwasserstoffe, wie beispielsweise Benzol, Toluol, Xylol, Chlorbenzol, Hexan, Cyclohexan, Chloroform; Ether, wie Diisopropylether, t-Butylmethylether, t-Amyl-methylether, Dioxan, Tetrahydrofuran oder Ethylenglykol-dimethyl- oder -diethylether; Ketone, wie Aceton, Butanon, Methyl-isopropyl-keton oder Methyl-isobutyl-keton; Nitrile, wie Acetonitril, Propionitril oder Butyronitril; Ester wie Essigsäure-methylester, -ethylester, -n- oder -i-propylester, -n-, -i-, -s- oder t-butylester.

Als bevorzugte Lösungsmittel für das erfindungsgemäße Verfahren seien Ether, wie Ethylenglykol-dimethylether und t-Amyl-methylether genannt.

Die Reaktionstemperaturen können bei der Durchführung des erfindungsgemäßen Verfahrens in einem größeren Bereich variiert werden. Im allgemeinen arbeitet man bei Temperaturen zwischen 80°C und 220°C, vorzugsweise bei Temperaturen zwischen 150°C und 200°C.

Das erfindungsgemäße Verfahren wird vorzugsweise unter erhöhtem Druck durchgeführt. Vorzugsweise wird im Druckbereich zwischen 1 und 100 bar, insbesondere zwischen 2 und 50 bar gearbeitet.

In einer bevorzugten Ausführungsform des erfindungsgemäßen Verfahrens wird das 6-Carboxy-1,2,4-triazin-dion der Formel (II) in einem aprotischen organischen Lösungsmittel suspendiert und in einem Autoklaven bis zum Ende der Umsetzung bei der zur Decarboxylierung erforderlichen Temperatur gerührt.

Die Aufarbeitung und Isolierung des Reaktionsproduktes kann auf übliche Weise durchgeführt werden. Beispielsweise wird abgesaugt und vom Filtrat das Lösungsmittel unter vermindertem Druck sorgfältig abdestilliert. Das als Rückstand verbleibende Produkt der Formel (I) kann im allgemeinen ohne weitere Reinigung weiter umgesetzt werden.

Die nach dem erfindungsgemäßen Verfahren herzustellenden Verbindungen der Formel (I) können als Zwischenprodukte zur Herstellung von endoparasitizid wirksamen Verbindungen eingesetzt werden (vgl. EP-A 330041).

### Herstellungsbeispiele:

### Beispiel 1

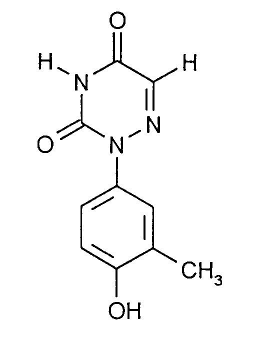

144,8 g (517 mMol) 6-Carboxy-2-(4-hydroxy-3-methylphenyl)-1,2,4-triazin-3,5(2H,4H)-dion werden in 870 ml Ethylenglykol-dimethylether suspendiert und die Mischung wird im verschlossenen Autoklaven 3 Stunden bei 200°C gerührt. Nach Abkühlen und Öffnen des Autoklaven wird abgesaugt und vom Filtrat das Lösungsmittel unter vermindertem Druck sorgfältig abdestilliert.

Man erhält 118,1 g (Gehalt: 79,3%, d.h. Ausbeute: 82,6% der Theorie) 2-(4-Hydroxy-3-methylphenyl)-1,2,4-triazin-3,5(2H,4H)-dion.

## Patentansprüche

1. Verfahren zur Herstellung von 1,2,4-Triazin-3,5-dionen der Formel (I) 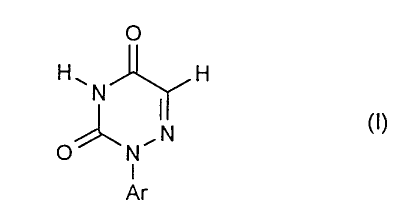 in welcher
Ar für jeweils gegebenenfalls substituiertes Aryl oder Heteroaryl steht,
indem man 6-Carboxy-1,2,4-triazindione der allgemeinen Formel (II) 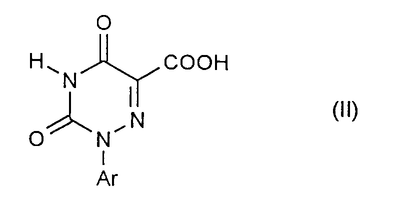 in welcher
Ar die oben angegebene Bedeutung hat,
bei Temperaturen zwischen 80°C und 220°C gegebenenfalls unter erhöhtem Druck umsetzt, dadurch gekennzeichnet, daß man die Umsetzung in aprotischen organischen Lösungsmitteln mit Siedepunkten zwischen 50°C und 150°C durchführt.
